# EUROPEAN PATENT SPECIFICATION

(11) **EP 3 878 987 B1**
(45) Date of publication and mention of the grant of the patent: **03.04.2024**
(21) Application number: 20869680.7
(22) Date of filing: 24.09.2020
(51) Int. Cl.: C22C 5/02, C22F 1/00, C22F 1/14, A61L 29/02, A61L 31/02

(54) **MEDICAL AU-PT-PD ALLOY**
MEDIZINISCHE AU-PT-PD-LEGIERUNG
ALLIAGE AU-PT-PD MÉDICAL

(30) Priority: 26.09.2019 JP 2019175204
(43) Date of publication of application: 15.09.2021
(73) Proprietor: Tanaka Kikinzoku Kogyo K.K., Tokyo 100-6422 (JP); Tokushima University, Tokushima-shi, Tokushima 770-8501 (JP)
(72) Inventor: OKUBO, Michimasa, Isehara-shi, Kanagawa 259-1146 (JP); GOTO, Kenji, Isehara-shi, Kanagawa 259-1146 (JP); TANAKA, Kunihiro, Isehara-shi, Kanagawa 259-1146 (JP); SHIRAISHI, Kojiro, Isehara-shi, Kanagawa 259-1146 (JP); SHIMA, Kunihiro, Isehara-shi, Kanagawa 259-1146 (JP); KATO, Yuya, Isehara-shi, Kanagawa 259-1146 (JP); HAMADA, Kenichi, Tokushima-shi, Tokushima 770-8501 (JP); HONDA, Eiichi, Tokushima-shi, Tokushima 770-8501 (JP); TAKEGAWA, Emi, Tokushima-shi, Tokushima 770-8501 (JP)
(74) Representative: Hoffmann Eitle
(86) International application number: PCT/JP2020/035902
(87) International publication number: WO 2021/060313

(56) References cited:
- WO-A1-2008/032370
- WO-A1-2015/093064
- JP-A- H08 316 263
- JP-A- H09 125 223
- JP-A- 2001 207 226
- JP-A- 2016 094 656
- JP-A- 2016 130 351
- US-A- 2 071 216
- US-A- 3 784 887
- US-A1- 2003 034 097
- US-B1- 6 248 190

## Description

### Technical Field

The present invention relates to a Au-Pt-Pd alloy suitable as a medical material which forms medical equipment such as an embolization coil or an embolization clip. In particular, the present invention relates to a medical material causing less artifacts in a magnetic field environment and having excellent mechanical properties, and a method for producing the medical material.

### Background Art

Attention is being paid to usefulness of endovascular treatment as a method for treating a cerebrovascular disorder such as brain aneurysm or subarachnoid hemorrhage. As medical equipment in treatment methods such as the endovascular treatment, various forms of medical equipment such as embolization coils, embolization clips, stents, catheters, and coils are applied. Since the medical equipment is a device which comes into direct contact with a human body and is embedded in the human body, the medical equipment is required to have biocompatibility and chemical stability (corrosion resistance). In addition, embolization coils etc. which are applied to the inside of pulsative and pulsatory blood vessels are required to have mechanical properties such as strength and a spring property. With consideration given to these demand characteristics, various metal materials such as Pt-W alloys (e.g. Pt-8 mass% W alloys), Ti alloys (e.g. Ti-6 mass% Al-4 mass% V alloys), and stainless steel (e.g. SUS 316L) have been applied.

In medical settings in recent years, diagnoses and treatments using magnetic resonance imaging diagnostic processors (MRI) have been extensively carried out, and impacts of the medical material in a magnetic field environment have been a concern. Examples of the characteristics of metal materials which are considered as a concern in the magnetic field environment include magnetic susceptibility. The reason why the magnetic susceptibility of the metal material is a concern is that the metal material causes magnetic susceptibility artifacts (false images) in MRI. The magnetic susceptibility artifact (hereinafter, referred to an "artifact") is a phenomenon in which a difference between the magnetic susceptibility of a metal in a magnetic field and the magnetic susceptibility of a biological tissue in a peripheral region of the metal causes a distortion in a MRI image. Generation of artifacts hampers accurate operations and diagnoses.

Here, many of the above-described proven medical metal materials have large magnetic susceptibility with respect to the magnetic susceptibility of biological tissues. In this connection, adjustment of parameters in an MRI apparatus, or the like has been performed as a conventional method for coping with artifacts from medical equipment in MRI. However, it can be hardly said that adjustment for the MRI apparatus is a fundamental solution against artifacts. In addition, MRI apparatuses have been directed to having a superhigh magnetic field in order to enhance the definition for securing accuracy of diagnosis etc. and enhance the speed. For apparatuses with a superhigh magnetic field, the artifact problem cannot be solved by conventional coping methods.

The present applicant has proposed a Au-Pt alloy containing a predetermined amount of Pt and Au as a balance for coping with the artifact problem with medical metal materials (Patent Document 1). The Au-Pt alloy is a metal material whose magnetic susceptibility is adjusted to fall within a suitable range by controlling a metal structure while alloying Pt with Au which is a diamagnetic metal.

Patent Document 2 relates to a hard precious metal alloy member composed of a gold alloy, which has a gold Au content of from 37.50 to 98.45 wt %, and contains a hardening additive in a range of not less than 50 ppm but less than 15,000 ppm, wherein the hardening additive is constituted of gadolinium Gd only, or gadolinium Gd and at least one element selected from the group consisting of rare-earth elements other than Gd, alkaline-earth elements, silicon Si, aluminum Al, and boron B.

Patent Document 3 relates to a body compatible stent formed of multiple filaments arranged in two sets of oppositely directed helical windings interwoven with one another in a braided configuration. Each of the filaments is a composite including a central core and a case surrounding the core. The core is formed of a radiopaque and relatively ductile material, in particular gold or a gold alloy. The outer case is formed of a relatively resilient material, e.g. a cobalt/chromium based alloy. The composite filaments are formed by a drawn filled tubing process in which the core is inserted into a tubular case of a diameter substantially more than the intended final filament diameter. The composite filament is cold-worked in several steps to reduce its diameter, and annealed between successive cold-working steps. After the final cold working step, the composite filament is formed into the desired shape and age hardened.

Patent Document 4 relates to relates to a process for making multilayer monolithic capacitors, using metallizations which can be cofired with reactive dielectric materials at elevated temperatures. The metallizations contain critical proportionate amounts of Pt and/or Au and Pd, and optionally Ag

Patent Document 5 relates to a bonding wire wherein the circumferential face of a core material composed of Au of 99.99% or higher purity is covered with an outer circumferential material composed of an Au alloy, and the diameter of the core material is set at 30% or higher and 90% or lower of the total diameter of a wire. As the Au alloy which constitutes the outer circumferential material, an alloy which contains one or more kinds out of, e.g. 0.05 to 0.5wt.% of Ru, 0.1 to 5.0wt.% of Pt, 0.1 to 5.0wt.% of Pd and 0.1 to 10wt.% of Ag and the residual part of which is composed of Au and unavoidable impurities can be used.

Patent Document 6 relates to an alloy that consists of gold, 15 to 25 wt% palladium and 0 to 10 wt% platinum so that the total content of palladium and platinum is ≥18 wt%, or consists of gold, 15 to 24.9 wt% palladium and 0 to 10 wt% platinum so that the total content of palladium and platinum is ≥ 18 wt%, and further contains one or more kinds of 0.1 to 3.0% zinc, 0.1 to 3.0% indium, 0.1 to 3.0% tin, 0.1 to 5.0% silver and 0.1 to 5.0% copper.

Patent Document 7 relates to the production and heat treatment of precious metal alloys containing more than 50% of gold and more particularly of such alloys the other components of which are platinum and palladium.

### Related Art Document

### Patent Document

Patent Document 1: JP 5582484 B2
Patent Document 2: US 2003/034097 A1
Patent Document 3: US 6 248 190 B1
Patent Document 4: US 3 784 887 A
Patent Document 5: JP H08 316263 A
Patent Document 6: JP 2001 207226 A
Patent Document 7: US 2 071 216 A

### Summary of the Invention

### Problems to be Solved by the Invention

A medical metal material including the above-described conventional Au-Pt alloy is one which can be referred to as "artifactless" as the magnetic susceptibility is extremely close to the magnetic susceptibility (-9 ppm (-9×10⁻⁶)) of water which is a main constituent of biological tissues. However, the alloy is inferior in mechanical characteristics to conventional materials, and it is difficult to apply the alloy to medical equipment. For example, for the above-described embolization coil, high strength and a spring property are required, and a Pt-8 mass% W alloy has been heretofore used. The Au-Pt alloy has lower mechanical properties as compared to the Pt-8 mass% W alloy, and is therefore difficult to apply to the embolization coils.

The present invention has been made in view of the above-described situations, and an object of the present invention is to provide a medical metal material which is probably used in a magnetic field environment with MRI etc. The medical metal material is a medical alloy material having preferred magnetic susceptibility to improve the artifact problem and having excellent mechanical properties.

### Means for Solving the Problems

The present inventors conducted studies for finding a material capable of solving the above-described problems. In the studies, the above-described Au-Pt alloy was used as a base, and Pd was added to this alloy as an additive for improvement of mechanical properties. On the other hand, Pd is a paramagnetic metal element, and therefore shifts the magnetic susceptibility of an alloy in a positive direction. Thus, excessive addition of Pd eliminates the artifactless characteristic of the Au-Pt alloy.

For a Au-Pt-Pd alloy obtained by adding Pd to the Au-Pt alloy, the present inventors adjusted an alloy composition and a metal structure, and examined an effect on magnetic susceptibility and mechanical properties. As a result, they found a range enabling exhibition of preferred characteristics, and in this way, attained the present invention.

Specifically, the present invention provides a medical Au-Pt-Pd alloy including Au, Pt, Pd, and inevitable impurities as defined in the claims. The medical Au-Pt-Pd alloy has an alloy composition inside a polygon (A1-A2-A3-A4) surrounded by straight lines connected at point A1 (Au: 53 atom%, Pt: 4 atom%, and Pd: 43 atom%), point A2 (Au: 70 atom%, Pt: 4 atom%, and Pd: 26 atom%), point A3 (Au: 69.9 atom%, Pt: 30 atom%, and Pd: 0.1 atom%), and point A4 (Au: 49.9 atom%, Pt: 50 atom%, and Pd: 0.1 atom%) in a Au-Pt-Pd ternary state diagram. In a metal structure on any cross-section, with respect to a composition of a Au-Pt-Pd alloy as a mother phase, at least one of a Au-rich phase which is an alloy phase having a Au content higher by 4 atom% or more than that of the mother phase and a Pt-rich phase which is an alloy phase having a Pt content higher by 4 atom% or more than that of the mother phase is distributed, and a total of an area ratio of the Au-rich phase and an area ratio of the Pt-rich phase is 1.5% or more and 25.4% or less.

As described above, the present inventive medical alloy material includes a Au-Pt-Pd alloy having an alloy composition within a certain composition range, and showing a metal structure having a Au-rich phase and a Pt-rich phase different in composition from a mother phase in the metal structure. Hereinafter, the constitutions of the present invention will be described in detail. In the following description, the Au-rich phase and the Pt-rich phase are sometimes referred to as separate phases.

### (A) Alloy composition

### (A-1) Composition range of essential elements

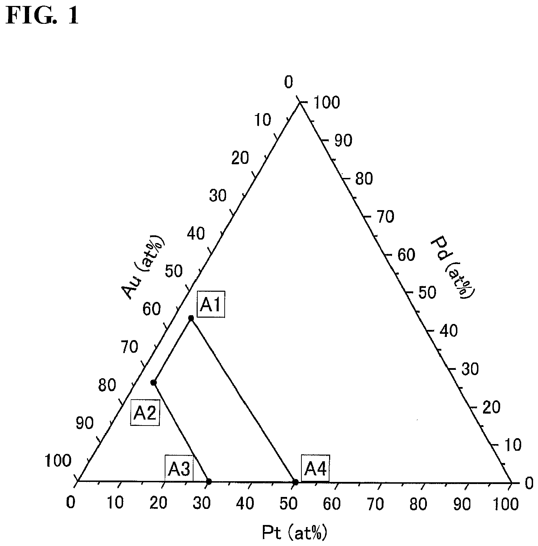

As described above, the present inventive medical Au-Pt-Pd alloy has an alloy composition inside a polygon (A1-A2-A3-A4) surrounded by straight lines connected at point A1 (Au: 53 atom%, Pt: 4 atom%, and Pd: 43 atom%), point A2 (Au: 70 atom%, Pt: 4 atom%, and Pd: 26 atom%), point A3 (Au: 69.9 atom%, Pt: 30 atom%, and Pd: 0.1 atom%), and point A4 (Au: 49.9 atom%, Pt: 50 atom%, and Pd: 0.1 atom%) in a Au-Pt-Pd ternary state diagram (Fig. 1). This composition range and a metal structure as described later enable the alloy to have a good balance in terms of magnetic susceptibility and mechanical properties. There remains a possibility that alloys having a composition outside the above-mentioned range is inferior in magnetic susceptibility and strength to conventional arts. Specifically, for magnetic susceptibility, alloys having a composition outside the above-mentioned range cause artifacts as easily as or more easily than the Pt-W alloy which is a conventional art. For strength, alloys having a composition outside the above-mentioned range have strength lower than that of the Au-Pt alloy which is a basic alloy of the present invention. In the following description, the composition range surrounded by point A1-point A2-point A3-point A4 is sometimes referred to as a "good region".

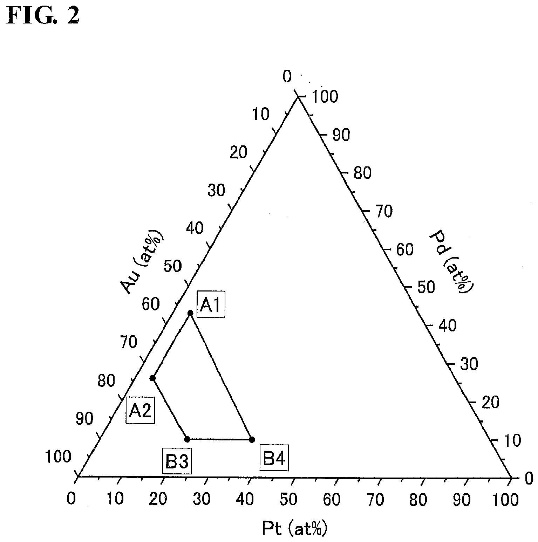

Preferably, the Au-Pt-Pd alloy having an alloy composition within the above-mentioned range has an alloy composition inside a polygon (A1-A2-B3-B4) surrounded by straight lines connected at point A1 (Au: 53 atom%, Pt: 4 atom%, and Pd: 43 atom%), point A2 (Au: 70 atom%, Pt: 4 atom%, and Pd: 26 atom%), point B3 (Au: 70 atom%, Pt: 20 atom%, and Pd: 10 atom%), and point B4 (Au: 55 atom%, Pt: 35 atom%, and Pd: 10 atom%) (Fig. 2). The composition enables formation of an alloy having a composition within a range more limited than the above-mentioned range and exhibiting more preferred magnetic susceptibility and mechanical strength. In the following description, the composition range surrounded by point A1-point A2-point B3-point B4 is sometimes referred to as a "better region".

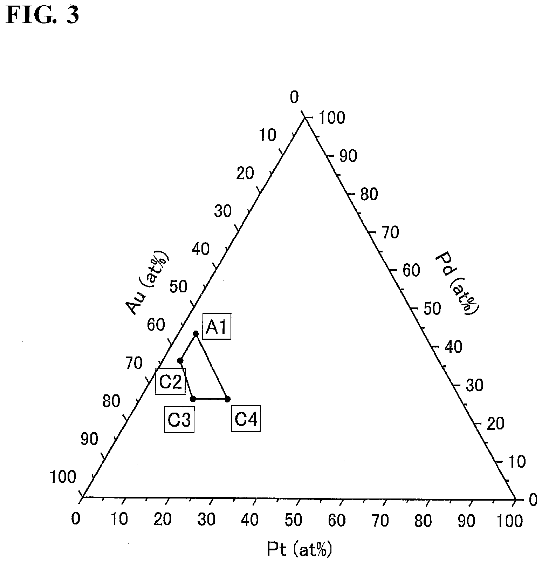

The Au-Pt-Pd alloy is particularly preferably an alloy having an alloy composition within the above-mentioned range inside a polygon (A1-C2-C3-C4) surrounded by straight lines connected at point A1 (Au: 53 atom%, Pt: 4 atom%, and Pd: 43 atom%), point C2 (Au: 60 atom%, Pt: 4 atom%, and Pd: 36 atom%), point C3 (Au: 62 atom%, Pt: 12 atom%, and Pd: 26 atom%), and point C4 (Au: 54 atom%, Pt: 20 atom%, and Pd: 26 atom%) (Fig. 3). The alloy produced based on the limited composition range can exhibit particularly preferred magnetic susceptibility and mechanical strength. The alloy having a composition within the above-mentioned range can be expected to have an artifact suppressing effect on a magnetic diagnosis apparatus such as a MRI whose magnetic field will increase in the future. In addition, the alloy having a composition within the above-mentioned range is particularly suitable as a constituent material of medical equipment required to have high strength and high elasticity, such as embolization coils. In the following description, the composition range surrounded by point A1-point C2-point C3-point C4 is sometimes referred to as a "best region".

### (A-2) Range of arbitrary elements and impurity elements

The present inventive alloy includes a tertiary alloy of Au, Pt, and Pd, and may contain a very small amount of additive elements. Specifically, the alloy may contain Ca and Zr. These additive elements have an effect of increasing the strength of the alloy, etc., and are contained in a total amount of 0 mass% or more and 0.5 mass% or less. The present inventive alloy may contain inevitable impurities. As the inevitable impurities, Ag, Co, Cr, Fe, Ir, Mg, Ni, Rh, Ru, Si, Sn, and Ti may be contained, and the total amount of the inevitable impurities contained is 0 ppm or more and 200 ppm or less. These additive elements and/or inevitable impurity elements replace a part of Au in the Au-Pt-Pd alloy.

### (B) Metal structure

### (B-1) Configuration of metal structure

The present inventive medical Au-Pt-Pd alloy is made to exhibit magnetic properties suitable for medical alloys by setting the composition within the above-mentioned range, and controlling a metal structure of an alloy having a composition within the range. This metal structure refers to a metal structure in which at least one of the Au-rich phase and the Pt-rich phase is distributed in the mother phase.

The mother phase refers to a phase of a Au-Pt-Pd alloy constituting a matrix of the metal structure, and is an alloy layer having a composition identical or substantially identical to the alloy composition of the Au-Pt-Pd alloy. Specifically, the mother phase is an alloy phase in which the content of each of Au and Pt is within the range of ±3 atom% with respect to the overall composition of the Au-Pt-Pd alloy. The mother phase is an alloy of an supersaturated solid solution obtained by subjecting a melted and cast alloy to homogenization treatment and solution treatment in a method for producing an alloy as described later.

The Au-rich phase is an alloy phase having a Au content higher by 4 atom% or more than the Au content of the mother phase. The Au-rich phase is basically a Au-Pt-Pd alloy. While having a Au content higher than that of the mother phase, the Au-rich phase has a Pt content lower than that of the mother phase and a Pd content close to that of the mother phase. The Pt content of the Au-rich phase is lower by 4 atom% or more than the Pt content of the mother phase. The Pd content of the Au-rich phase is within the range of ±2 atom% with respect to the Pd content of the mother phase.

The Pt-rich phase is an alloy phase having a Pt content higher by 4 atom% or more than the Pt content of the mother phase. The Pt-rich phase is basically a Au-Pt-Pd alloy. While having a Pt content higher than that of the mother phase, the Pt-rich phase has a Au content lower than that of the mother phase and a Pd content close to that of the mother phase. The Au content of the Pt-rich phase is lower by 4 atom% or more than the Au content of the mother phase. The Pd content of the Au-rich phase is within the range of ±2 atom% with respect to the Pd content of the mother phase.

In the present invention, a material structure is presented in which at least one of the Au-rich phase and the Pt-rich phase which are separate phases is distributed in the mother phase.

As described above, the present inventive Au-Pt-Pd alloy has preferred magnetic characteristics (volume magnetic susceptibility) because the alloy composition (contents of Au, Pt, and Pd) is within a certain range. The reason why the Au-rich phase and the Pt-rich phase are distributed in the present invention is that these separate phases cooperate with the adjustment effect of the alloy composition to optimize magnetic characteristics. In the present invention, the Au-rich phase having a high Au content tends to have magnetic susceptibility on a negative side with respect to the magnetic susceptibility of the mother phase. On the other hand, the Pt-rich phase having a high Pt content tends to have magnetic susceptibility on a positive side. The magnetic characteristics of these separate phases different in magnetic susceptibility act on the characteristics of the mother phase to optimize the magnetic characteristics of the alloy.

### (B-2) Area ratio of separate phase

In the present invention, the total of the area ratio of the Au-rich phase and the area ratio of the Pt-rich phase is 1.5% or more and 25.4% or less in the metal structure on any cross-section. When the area ratio of the separate phases is less than 1.5%, the alloy is substantially a single-phase alloy, and an adjustment effect of the separate phase is not obtained. Here, the alloy is comparable in magnetic susceptibility to conventional arts. Additionally, it is difficult to uniformly disperse separate phases when the area ratio of the separate phases exceeds 25.4%. Since the Au-rich phase and the Pt-rich phase each have magnetic susceptibility different from that of the mother phase, nonuniform distribution of the separate phases may hamper stabilization of the magnetic characteristics of the alloy.

It is not necessary to separately define the area ratio of the Au-rich phase and the area ratio of the Pt-rich phase. All the Au-rich phase (Pt-rich phase) distributed in the mother phase does not necessarily have the same composition. In the Au-rich phase (Pt-rich phase), the relation between the Au content (Pt content) and the magnetic susceptibility is not a simple proportional linear relation. Thus, it is difficult to precisely discriminate and define the area ratio of the Au-rich phase from the area ratio of the Pt-rich phase. The effect of the Au-rich phase and the Pt-rich phase is determined based on the total amount of the phases by magnetic susceptibility as described later.

The total area ratio of the separate phases (Au-rich phase and Pt-rich phase) can vary depending on both of factors which are the alloy composition (good region, better region, or best region) and the production conditions (working step and heat treatment step as described later). The magnetic characteristics and the strength of the alloy vary depending on both the alloy composition and the total area ratio of the separate phases. The characteristics are not determined by only one of the alloy composition and the total area ratio of the separate phases. For the alloy composition of the good region, an alloy may be obtained in which the separate phases have a total area ratio of 11% or more and 25.4% or less. For the alloy composition of the better region and the best region, an alloy tends to be obtained in which the separate phases have a relatively low total area ratio of 1.5% or more and 11% or less.

In the present invention, the area ratio refers to an area ratio based on the observation visual field in observation of a material structure on any cross-section. As indicated by "any cross-section", the cut section or the cutting direction during observation is not specified. In addition, in the present inventive Au-Pt-Pd alloy, the area ratio of a predetermined separate phase is determined, but an observation visual field is not selected in such a manner as to intentionally exclude the separate phase. The size of the observation visual field is preferably in a range of 10,000 to 50,000 µm². For calculation of the area ratio of separate phases in the alloy, area ratios for cross-sections at a plurality of arbitrary positions may be measured, followed by calculating an average of the area ratios. For measurement of the area ratio of the separate phases, image processing software or the like may be used.

### (B-3) Form of separate phase

The Au-rich phase and the Pt-rich phase described above may be distributed alone, or distributed while forming a mixed phase in which the Au-rich phase and the Pt-rich phase aggregate or continue. When a region formed by the mixed phase of the Au-rich phase and the Pt-rich phase is referred to as a separate region in the present invention, examples of the form of the separate region include the following.

### (B-3-1) Separate region A

The separate region A is a relatively regular region including a mixed phase which contains one or more Au-rich phases and one or more Pt-rich phases and which is formed by deposition of the phases in a layered form. The separate region A is often present in proximity to crystal grain boundaries of the mother phase in the cross-sectional structure of the alloy. The separate region A is formed by deposition of the Au-rich phase and the Pt-rich phase along crystal grain boundaries. However, Au-rich phases and Pt-rich phases do not necessarily deposit alternately one by one. Continuation of Au-rich phases or Pt-rich phases may be followed by deposition of the other phases. In addition, in the separate region A, the Au-rich phase and the Pt-rich phase extend in a direction orthogonal to crystal grain boundaries, and the region often has a width of 0.5 µm or more and 20 µm or less.

### (B-3-1) Separate region B

The separate region B is an island-shaped phase including at least one of the Au-rich phase and the Pt-rich phase. The separate region B can take a variety of forms. The separate region B is a mixed phase of the Au-rich phase and the Pt-rich phase. The separate region B may be in a regular layered or striped form like the separate region A, or both the phases may be mixed in a random shape. One phase may be scattered inside the other phase. The separate region B can take various mixed forms. The separate region may be composed only one of the Au-rich phase or the Pt-rich phase. The separate region B tends to be scattered inside crystal grains of the mother phase. The separate region B often forms a region having a grain size of 0.5 µm or more and 20 µm or less in terms of an equivalent circle diameter.

The two separate regions are naturally formed by precipitation of the Au-rich phase and the Pt-rich phase. The Au-rich phase and the Pt-rich phase are precipitated during aging heat treatment in a method for producing an alloy as described later. Since these separate regions are easily generated in a region with high energy in the mother phase, the separate regions are formed preferentially on the crystal grain boundaries and the outer surface of the mother phase, and tend to develop in the form of the separate region A. In addition, only the separate region A or only the separate region B may be precipitated, or both the separate regions may be precipitated. Impacts of the form of these separate regions on magnetic properties and mechanical properties cannot be definitely affirmed, but cannot be denied.

### (C) Magnetic and mechanical properties of alloy

The present inventive Au-Pt-Pd alloy has a potential of exhibiting magnetic susceptibility enabling suppression of artifacts, and mechanical propertied required as medical equipment. As specific values of the magnetic and mechanical properties of the present inventive Au-Pt-Pd alloy, the volume magnetic susceptibility is -32 ppm or more and 60 ppm or less, and the Young's modulus is 100 GPa or more. As preferred values of the magnetic and mechanical properties, the magnetic susceptibility is -32 ppm or more and 30 ppm or less, and the Young's modulus is 110 GPa or more. Particularly preferably, the magnetic susceptibility is -20 ppm or more and 0 ppm or less, and the Young's modulus is 130 GPa or more. In addition, as described above, the magnetic properties and the mechanical properties of the present inventive Au-Pt-Pd alloy can vary basically depending on both the alloy composition and the material structure (e.g. total area ratio of separate phases).

### (D) Method for producing present inventive Au-Pt-Pd alloy

A method for producing the present inventive Au-Pt-Pd alloy will now be described. The method for producing the present inventive alloy includes the steps of: melting and casting a mother alloy having a composition within the above-mentioned range; subjecting the mother alloy to homogenization treatment; subjecting to plastic working the mother alloy subjected to the homogenization treatment; subjecting to solution treatment the alloy subjected to plastic working; and performing aging heat treatment for controlling a metal structure. These steps will be described.

Methods and conditions in melting and casting methods can be applied to the melting and casting step for producing the mother alloy. For adjustment of the alloy composition, raw metals of Au, Pt, and Pd can be mixed at the above-mentioned composition, and melted and cast by arc melting, high-frequency melting or the like to produce an ingot of the mother alloy.

The homogenization treatment is a treatment step for homogenizing the metal structure by heating the mother alloy produced in the melting and casting step. The homogenization treatment is important in formation of a solid solution by subsequent solution treatment and optimization of a precipitated state of separate phases by age treatment. In the present invention, Pd is added to a conventional Au-Pt alloy, and as the number of constituent elements increases, atom transfer of the constituent elements by the homogenization treatment becomes more important. In addition, by performing the homogenization treatment, processability in plastic working in the step can be improved. As a condition for the homogenization treatment, the treatment temperature is 1,000°C or higher and 1,200°C or lower. In the homogenization heat treatment, the higher the temperature, the more efficient. This is because in a Au-Pt-Pd ternary system, there is a composition range where the alloy melts at 1,200°C. The treatment time is preferably 1 hour or more and 48 hours or less.

A plastic working step and a solution treatment step are carried out on the mother alloy after the homogenization treatment, and the alloy is formed into a single-phase supersaturated solid solution on a temporary basis. The present inventive alloy has a configuration in which a predetermined separate phase is precipitated in a metal structure. This separate phase is generated in aging heat treatment which is the last step, and for generating separate phases in a preferred state, treatment with a solution treatment step added after the plastic working step is required together with the above-described homogenization treatment.

The plastic working step refers to a step for introducing a drive force for generation of a solid solution by solution treatment. In addition, the plastic working step serves as a step of preliminarily adjusting a size and a shape before forming the alloy into a final shape. The plastic working is preferably cold working in which the working temperature is ordinary temperature or hot working in which the working temperature is 400°C or lower. The working rate is preferably 30% or more and 90% or less. However, the form of the working is not limited, and various forms of working methods such as swaging, rolling, casting, wire drawing, and extrusion are applied.

For the heat treatment in the solution treatment step, the plastically-worked mother alloy is heated to 1,000°C or higher and 1 ,200°C or lower. The heating time during heat is 1 to 24 hours. In addition, it is preferable to adopt rapid cooling as cooling after heating and to put the alloy in a cooling medium such as water within 3 seconds or less after the heating. The number of treatments for single-phasing by combining the above plastic working and solution treatment may be one, or two or more.

The supersaturated solid solution alloy obtained in the manner described above generates separate phases by aging heat treatment to form the alloy of the present invention. The temperature in the aging heat treatment is in a range of 400 to 800°C. The heat treatment time is preferably 0.5 to 48 hours.

In the present invention, the supersaturated solid solution alloy may be subjected to plastic working before aging heat treatment. This is because when by plastic working before the aging, working strain is made to remain in the supersaturated solid solution alloy, and the strain energy is utilized as a drive force for phase separation during the age treatment, separate phases in a preferred state may be formed. The plastic working before age treatment is preferably cold working at one time (one pass) or more, and may be any of working modes such as swaging, rolling, casting, wire drawing, and extrusion. The working rate per pass is preferably 12 to 18%. When working at a plurality of passes is performed, the total working rate is preferably 50% or more. With the plastic working step as a final working step, the alloy may be formed into a size and a shape available for various kinds of medical equipment. The plastically-worked mother alloy can be subjected to the above-described age treatment to form the alloy of the present invention.

By the age treatment, separate phases are generated to produce the present inventive Au-Pt-Pd alloy. This alloy may be further subjected to plastic working.

Examples of the medical equipment to which the above-described present inventive medical Au-Pt-Pd alloy is suitably applied include various forms of medical equipment such as coils such as embolization coils, embolization clips, stents such as flow diverter stents and stent retrievers, and catheters such as balloon catheters.

For application to such medical equipment, the shape of the alloy of the present invention is not limited, and the alloy is processed into various forms such as those of wire materials, bar materials, square materials, hollow materials, and plate materials. For example, for the embolization coil, the alloy is processed into a wire material, and then molded by a winding machine to prepare a coil. The stent is prepared by using a knitting machine to knit the alloy processed into a wire material. The stent retriever is prepared by molding the alloy processed into a pipe material or a tube material. The present inventive Au-Pt-Pd alloy has good processability, and can be processed into various shapes as described above.

### Advantageous Effects of the Invention

As described above, the present inventive Au-Pt-Pd alloy has magnetic susceptibility suitable as a medical metal material, and is improved in terms of the artifact problem in magnetic diagnosis equipment such as a MRI. The alloy is excellent in mechanical properties, and has good strength, operability, and durability when used in various kinds of medical equipment.

### Brief Description of the Drawings

Fig. 1 is a ternary state diagram of a Au-Pt-Pd alloy, which also shows a composition range (A1-A2-A3-A4: good region) of the present inventive alloy.
Fig. 2 is a ternary state diagram of a Au-Pt-Pd alloy, which also shows the composition range (A1-A2-B3-B4: better region) of the present inventive alloy.
Fig. 3 is a ternary state diagram of a Au-Pt-Pd alloy, which also shows the composition range (A1-C2-C3-C4: best region) of the present inventive alloy.
Fig. 4 is a ternary state diagram showing a composition of a Au-Pt-Pd alloy produced in an embodiment.
Fig. 5 is a diagram showing a metal structure of an alloy of No. 7.

### Description of Embodiments

Hereinafter, an embodiment of the present invention will be described. In this embodiment, Au-Pt-Pd alloys of various compositions were produced, and evaluated for magnetic characteristics and mechanical characteristics.

For the Au-Pt-Pd alloys, raw metals of pure Au, pure Pt, and pure Pd with a purity of 99.99% were weighed to various compositions, and melted at a high frequency and cast into an alloy ingot (crucible: zirconia crucible, mold: water-cooled Cu mold, maximum power during melting: 2.5 kW). A mother alloy ingot of 7 mm (diameter) × 65 mm was produced by the melting and casting step.

Next, the mother alloy was subjected to homogenization treatment in which the alloy was heated in an Ar atmosphere at 1,100°C for 1 hour. After the heating, the mother alloy was cooled with water.

The homogenization-treated mother alloy was subjected to plastic working and solution treatment. In the plastic working step, swaging was performed at ordinary temperature, and the ingot with a diameter of 7 mm was reduced in diameter to a diameter of 4 mm in increments of 0.5 to 1 mm. The processed mother alloy was heated in an Ar atmosphere at a temperature of 1,100°C for 12 hours, and then rapidly cooled to perform solution treatment.

After the solution treatment and before age treatment, the mother alloy with a diameter of 4 mm was subjected to wire drawing at a working rate of 15% per pass until the diameter was 3 mm. In the age treatment, the mother alloy was heated in an Ar atmosphere at a temperature of 600°C for 1 hour, and then rapidly cooled. By the above steps, a Au-Pt-Pd alloy wire material was produced. For some alloys (Nos. 16 and 17 in Table 1 below), the heat treatment temperature was 300°C.

For the Au-Pt-Pd alloys produced in this embodiment, cross-sections were observed by SEM to examine metal structures. In the structure observation, a sample obtained by cutting the wire material at any position was polished into a mirror surface state, and subjected to ion milling to make the surface state easily observable. The sample was then observed by SEM.

In observation of separate phases which are an Au-rich phase and a Pt-rich phase, the Au-rich phase and the Pt-rich phase were identified with respect to a matrix phase by SEM-EDX composition analysis (accelerating voltage: 15 kV). In addition, when there was a sample in which it was difficult to identify the Au-rich phase and the Pt-rich phase, a surface of the sample was observed by EPMA (accelerating voltage: 15 kV), and mapping was performed to identify the phases. The area ratios of the Au-rich phase and the Pt-rich phase were determined by image evaluation. For the image evaluation, the area ratios of the separate phases were calculated by use of the crystal grain evaluation tool: Grain Expert which is commercially available image analysis software (Leica Application Suite manufactured by Leica).

Further, the Au-Pt-Pd alloys produced in this embodiment were subjected to volume magnetic susceptibility measurement, processability evaluation, and mechanical property evaluation. For the volume magnetic susceptibility measurement, a sample of 3 mm (diameter) × 8 mm was prepared, and volume magnetic susceptibility (Xv) was measured at room temperature (25°C) by use of a high-sensitivity small magnetic balance (MSB-AUTO).

For the processability, whether or not breakage occurred was evaluated in formation of a wire material with a diameter of 1 mm by subjecting a wire material (diameter: 3 mm) to wire drawing at a working rate of 10% per pass. For the mechanical properties, a sample with a diameter of 1 mm was set with a chuck-to-chuck distance of 100 mm in a tensile tester, and a tension test was conducted at a cross head speed of 1 mm/min to measure a Young's modulus.

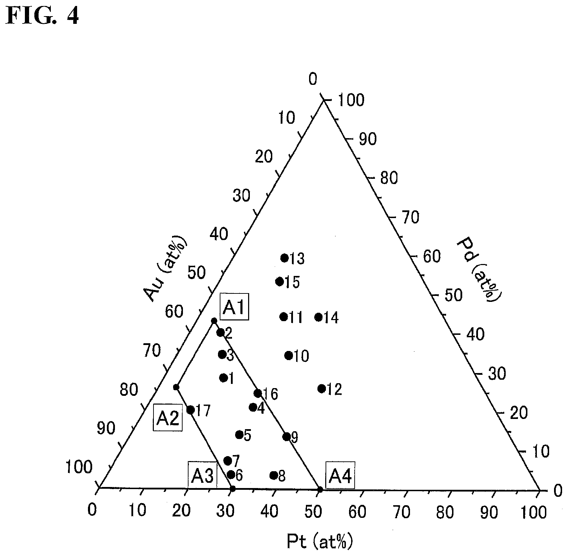

Table 1 shows the evaluation results of various Au-Pt-Pd alloys produced in this embodiment. In addition, the compositions of the Au-Pt-Pd alloys produced in this embodiment are shown in a ternary state diagram of Fig. 4.

**[Table 1]**

| No. | Composition (at%) | | | Total area ratio of separate phases (%) | Xv (ppm) | Young's modulus (GPa) | Processability |
|---|---|---|---|---|---|---|---|
| | Au | Pt | Pd | | | | |
| 1 | Balance | 13.5 | 28.5 | 1.9 | -9 | 138 | ○ |
| 2 | | 7 | 40 | 1.5 | -2 | 137 | ○ |
| 3 | | 10.2 | 34.4 | 10.2 | -5 | 137 | ○ |
| 4 | | 24 | 21 | 10.4 | 14 | 113 | ○ |
| 5 | | 24.5 | 13.9 | 4.7 | 5 | 122 | ○ |
| 6 | | 27.8 | 3.7 | 12.8 | -10 | 106 | ○ |
| 7 | | 25.3 | 7.2 | 22.5 | -7 | 109 | ○ |
| 8 | | 37.6 | 3.6 | 11.3 | 33 | 118 | ○ |
| 9 | | 35.5 | 13.5 | 25.4 | 41 | 122 | ○ |
| 10* | Balance | 25.4 | 34.3 | 0.1 | 61 | 146 | ○ |
| 11* | | 19.3 | 44.2 | 0.5 | 79 | 152 | ○ |
| 12* | | 37.2 | 26 | 0.5 | 77 | 148 | ○ |
| 13* | | 11.7 | 59.3 | 0.5 | 138 | 174 | ○ |
| 14* | | 27.2 | 44.2 | 0.1 | 119 | 157 | ○ |
| 15* | | 13.7 | 53.2 | 1.8 | 109 | 135 | ○ |
| 16* | | 23.3 | 24.6 | 0.9 | 61 | 137 | ○ |
| 17* | | 10.2 | 20.2 | 0.4 | -34 | 117 | ○ |

| | | | | | | | |
|---|---|---|---|---|---|---|---|
| * = comparative, not in accordance with the invention | | | | | | | |

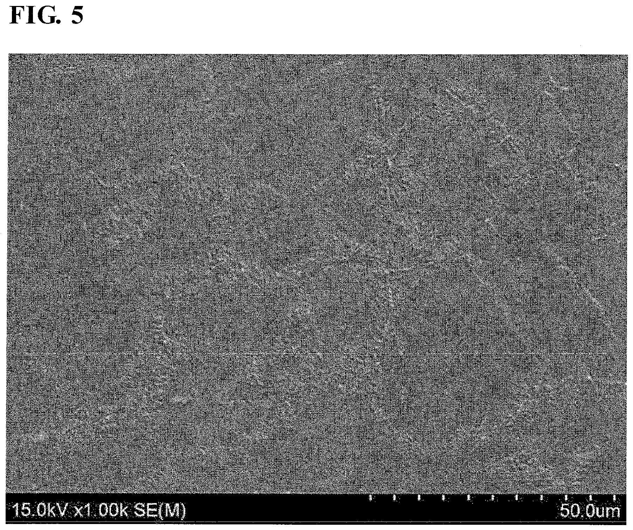

It will be apparent from Table 1 that in a region surrounded by point A1-point A2-point A3-point A4 (good region) in the ternary state diagram specified in the present application, the volume magnetic susceptibility of the Au-Pt-Pd alloy is within a range of -32 ppm or more and 60 ppm or less, and the Young's modulus is 100 GPa or more (Nos. 1 to 9). Fig. 5 is a photograph showing a metal structure of the alloy of No. 7. Separate phases deposited in a layered form are observed in proximity to a crystal grain boundary of the mother phase.

Au-Pt-Pd alloys with an alloy composition range in a better region or a best region narrower than the good region exhibits a more preferred volume magnetic susceptibility and Young's modulus (Nos. 1 to 5). The alloys of Nos. 1 to 3 have a particularly good volume magnetic susceptibility and Young's modulus, and the alloy of No. 3 is an alloy which has a volume magnetic susceptibility of - 5 ppm, so as to be artifactless.

On the other hand, alloys with a composition outside the composition range specified in the present invention tend to have a low area ratio of separate phases (Au-rich phase and Pt-rich phase) and a volume magnetic susceptibility of more than 60 ppm (Nos. 11 to 14). In addition, when the composition is outside the composition range, the volume magnetic susceptibility is not appropriate even through a separate phase area ratio of 1.5% or more (No. 15). In the Au-Pt-Pd alloy covered by the present invention, it is first considered necessary to optimize the composition range.

Indeed, even alloys with a composition in the composition range specified in the present application have a small area ratio of the Au-rich phase and the Pt-rich phase due to insufficient precipitation of these phases if heat treatment conditions during production are not appropriate (Nos. 16 and 17). These alloys cannot exhibit minimum required volume magnetic susceptibility (-32 ppm or more and 60 ppm or less).

Any alloy composition (good region, better region, or best region) enables optimization of only magnetic properties or mechanical properties. For example, alloys in which only the magnetic susceptibility is an optimum value (-20 ppm or more and 0 ppm or less) can be produced even if the composition is within the good region that is the widest range (Nos. 6 and 7). These alloys have a relatively low Young's modulus, and even such alloys are useful for applications in which the magnetic susceptibility is considered important. In use of the present inventive Au-Pt-Pd alloy, it may be preferable to consider both the alloy composition and the total area ratio of separate phases while giving a top priority to the required volume magnetic susceptibility and Young's modulus.

### Industrial Applicability

The present inventive medical Au-Pt-Pd alloy is suitable as a constituent material for medical equipment which is used in a magnetic field environment. The alloy of the present invention is capable of coping with the artifact problem, and has mechanical properties required for various kinds of medical equipment. The present invention can be expected to be applied to various kinds of medical equipment such as coils such as embolization coils, stents, catheters, and guide wires.

## Claims

1. A medical Au-Pt-Pd alloy consisting of Au, Pt, and Pd, a total amount of 0 mass% or more and 0.5 mass% or less of Ca and Zr as optional additive elements, and inevitable impurities, wherein
the medical Au-Pt-Pd alloy has an alloy composition inside a polygon (A1-A2-A3-A4) surrounded by straight lines connected at point A1 (Au: 53 atom%, Pt: 4 atom%, and Pd: 43 atom%), point A2 (Au: 70 atom%, Pt: 4 atom%, and Pd: 26 atom%), point A3 (Au: 69.9 atom%, Pt: 30 atom%, and Pd: 0.1 atom%), and point A4 (Au: 49.9 atom%, Pt: 50 atom%, and Pd: 0.1 atom%) in a Au-Pt-Pd ternary state diagram,
the medical Au-Pt-Pd alloy comprises a total amount of 0 ppm or more and 200 ppm or less of Ag, Co, Cr, Fe, Ir, Mg, Ni, Rh, Ru, Si, Sn, and Ti as inevitable impurities, and
the additive elements and inevitable impurity elements replace a part of Au in the Au-Pt-Pd alloy,
**characterized in that**
in a metal structure on any cross-section,
with a composition of a mother phase Au-Pt-Pd alloy as a criterion, at least one of a Au-rich phase which is an alloy phase having a Au content higher by 4 atom% or more than that of the mother phase and a Pt-rich phase which is an alloy phase having a Pt content higher by 4 atom% or more than that of the mother phase is distributed, and a total of an area ratio of the Au-rich phase and an area ratio of the Pt-rich phase is 1.5% or more and 25.4% or less, and
the medical Au-Pt-Pd alloy has a volume magnetic susceptibility of -32 ppm or more and 60 ppm or less and a Young's modulus of 100 GPa or more,
wherein the area ratio refers to one based on the observation visual field of a material structure on an arbitrary cross-section and is determined as described in the description, the volume magnetic susceptibility is determined with a magnetic balance as described in the description, and the Young's modulus is determined with a tensile tester as described in the description.

2. The medical Au-Pt-Pd alloy according to claim 1, which has an alloy composition within the range inside a polygon (A1-A2-B3-B4) surrounded by straight lines connected at point A1 (Au: 53 atom%, Pt: 4 atom%, and Pd: 43 atom%), point A2 (Au: 70 atom%, Pt: 4 atom%, and Pd: 26 atom%), point B3 (Au: 70 atom%, Pt: 20 atom%, and Pd: 10 atom%), and point B4 (Au: 55 atom%, Pt: 35 atom%, and Pd: 10 atom%) in the Au-Pt-Pd ternary state diagram.

3. The medical Au-Pt-Pd alloy according to claim 1, which has an alloy composition within the range inside a polygon (A1-C2-C3-C4) surrounded by straight lines connected at point A1 (Au: 53 atom%, Pt: 4 atom%, and Pd: 43 atom%), point C2 (Au: 60 atom%, Pt: 4 atom%, and Pd: 36 atom%), point C3 (Au: 62 atom%, Pt: 12 atom%, and Pd: 26 atom%), and point C4 (Au: 54 atom%, Pt: 20 atom%, and Pd: 26 atom%) in the Au-Pt-Pd ternary state diagram.

4. A method for producing the medical Au-Pt-Pd alloy defined in any one of claims 1 to 3, comprising the steps of:
melting and casting a mother alloy of the Au-Pt-Pd alloy;
heating the mother alloy at a temperature of 1,000°C or higher and 1,200°C or lower to perform homogenization treatment;
subjecting the homogenization-treated mother alloy to plastic working;
subjecting the plastically-worked alloy to solution treatment by heating to 1000°C or higher and 1200°C or lower for 1 to 24 hours; and
performing aging heat treatment in which the alloy is heated at 400 to 800°C for controlling a metal structure.

5. The method for producing the medical Au-Pt-Pd alloy according to claim 4, comprising the step of subjecting the solution-treated mother alloy to plastic working before the aging heat treatment.

6. A medical device comprising the medical Au-Pt-Pd alloy defined in any one of claims 1 to 3.

7. The medical device according to claim 6, wherein the medical device is one of a stent, a catheter, an embolization coil, an embolization clip, and a guide wire.

## Patentansprüche

1. Medizinische Au-Pt-Pd-Legierung, bestehend aus Au, Pt und Pd, einer Gesamtmenge von 0 Masse-% oder mehr und 0,5 Masse-% oder weniger von Ca und Zr als optionale Zusatzelemente und unvermeidlichen Verunreinigungen, wobei
die medizinische Au-Pt-Pd-Legierung eine Legierungszusammensetzung innerhalb eines Polygons (A1-A2-A3-A4) aufweist, das von Geraden umgeben ist, die am Punkt A1 (Au: 53 Atom-%, Pt: 4 Atom-% und Pd: 43 Atom-%), Punkt A2 (Au: 70 Atom-%, Pt: 4 Atom-% und Pd: 26 Atom-%), Punkt A3 (Au: 69,9 Atom-%, Pt: 30 Atom-% und Pd: 0,1 Atom-%), Punkt A4 (Au: 49,9 Atom-%, Pt: 50 Atom-% und Pd: 0,1 Atom-%) in einem ternären Au-Pt-Pd-Zustandsdiagramm verbunden sind,
die medizinische Au-Pt-Pd-Legierung eine Gesamtmenge von 0 ppm oder mehr und 200 ppm oder weniger von Ag, Co, Cr, Fe, Ir, Mg, Ni, Rh, Ru, Si, Sn und Ti als unvermeidliche Verunreinigungen umfasst und
die Zusatzelemente und die unvermeidlichen Verunreinigungselemente einen Teil von Au in der Au-Pt-Pd-Legierung ersetzen,
**dadurch gekennzeichnet, dass**
in einer Metallstruktur über irgendeinen Querschnitt mit einer Zusammensetzung einer Mutterphasen-Au-Pt-Pd-Legierung als Kriterium mindestens eine von einer Au-reichen Phase, die eine Legierungsphase mit einem Au-Gehalt ist, der um 4 Atom-% oder mehr höher als der der Mutterphase ist, und einer Pt-reichen Phase, die eine Legierungsphase mit einem Pt-Gehalt ist, der um 4 Atom-% oder mehr höher als der der Mutterphase ist, verteilt ist und eine Summe eines Flächenanteils der Au-reichen Phase und eines Flächenanteils der Pt-reichen Phase 1,5 % oder mehr und 25,4 % oder weniger beträgt und
die medizinische Au-Pt-Pd-Legierung eine magnetische Volumensuszeptibilität von -32 ppm oder mehr und 60 ppm oder weniger und einen Youngschen Modul von 100 GPa oder mehr aufweist,
wobei sich der Flächenanteil auf einen solchen bezieht, der auf dem Betrachtungssichtfeld einer Materialstruktur über einen beliebigen Querschnitt basiert und so, wie in der Beschreibung beschrieben, bestimmt wird, die magnetische Volumensuszeptibilität mit einer Magnetwaage so, wie in der Beschreibung beschrieben, bestimmt wird und der Youngsche Modul mit einem Zugprüfgerät so, wie in der Beschreibung beschrieben, bestimmt wird.

2. Medizinische Au-Pt-Pd-Legierung gemäß Anspruch 1, die eine Legierungszusammensetzung im Bereich innerhalb eines Polygons (A1-A2-B3-B4) aufweist, das von Geraden umgeben ist, die am Punkt A1 (Au: 53 Atom-%, Pt: 4 Atom-% und Pd: 43 Atom-%), Punkt A2 (Au: 70 Atom-%, Pt: 4 Atom-% und Pd: 26 Atom-%), Punkt B3 (Au: 70 Atom-%, Pt: 20 Atom-% und Pd: 10 Atom-%) und Punkt B4 (Au: 55 Atom-%, Pt: 35 Atom-% und Pd: 10 Atom-%) in dem ternären Au-Pt-Pd-Zustandsdiagramm verbunden sind.

3. Medizinische Au-Pt-Pd-Legierung gemäß Anspruch 1, die eine Legierungszusammensetzung im Bereich innerhalb eines Polygons (A1-C2-C3-C4) aufweist, das von Geraden umgeben ist, die am Punkt A1 (Au: 53 Atom-%, Pt: 4 Atom-% und Pd: 43 Atom-%), Punkt C2 (Au: 60 Atom-%, Pt: 4 Atom-% und Pd: 36 Atom-%), Punkt C3 (Au: 62 Atom-%, Pt: 12 Atom-% und Pd: 26 Atom-%) und Punkt C4 (Au: 54 Atom-%, Pt: 20 Atom-% und Pd: 26 Atom-%) in dem ternären Au-Pt-Pd-Zustandsdiagramm verbunden sind.

4. Verfahren zur Herstellung der in einem der Ansprüche 1 bis 3 definierten medizinischen Au-Pt-Pd-Legierung, umfassend die Schritte:
Schmelzen und Gießen einer Mutterlegierung aus der Au-Pt-Pd-Legierung,
Erwärmen der Mutterlegierung bei einer Temperatur von 1.000°C oder höher und 1.200°C oder niedriger, um eine Homogenisierungsbehandlung durchzuführen,
Unterziehen der homogenisierungsbehandelten Mutterlegierung einer plastischen Bearbeitung,
Unterziehen der plastisch bearbeiteten Legierung einer Lösungsbehandlung durch Erwärmen auf 1.000°C oder höher und 1.200°C oder niedriger für 1 bis 24 Stunden und
Durchführen einer Alterungswärmebehandlung, in der die Legierung bei 400 bis 800°C zur Regulierung einer Metallstruktur erwärmt wird.

5. Verfahren zur Herstellung der medizinischen Au-Pt-Pd-Legierung gemäß Anspruch 4, umfassend den Schritt zum Unterziehen der lösungsbehandelten Mutterlegierung einer plastischen Bearbeitung vor der Alterungswärmebehandlung.

6. Medizinische Vorrichtung, umfassend die in einem der Ansprüche 1 bis 3 definierten medizinische Au-Pt-Pd-Legierung.

7. Medizinische Vorrichtung gemäß Anspruch 6, wobei die medizinische Vorrichtung eines von einem Stent, einem Katheter, einer Embolisationsspule, einem Embolisationsclip und einem Führungsdraht ist.

## Revendications

1. Alliage Au-Pt-Pd médical consistant en Au, Pt et Pd, une quantité totale de 0 % en masse ou plus et 0,5 % en masse ou moins de Ca et Zr en tant qu'éléments additifs optionnels, et en impuretés inévitables, dans lequel
l'alliage Au-Pt-Pd médical présente une composition d'alliage à l'intérieur d'un polygone (A1-A2-A3-A4) entouré de lignes droites reliées au niveau du point A1 (Au : 53 % atomique, Pt : 4 % atomique, et Pd : 43 % atomique), du point A2 (Au : 70 % atomique, Pt : 4 % atomique, et Pd : 26 % atomique), du point A3 (Au : 69,9 % atomique, Pt: 30 % atomique, et Pd : 0,1 % atomique) et du point A4 (Au : 49,9 % atomique, Pt: 50 % atomique, et Pd : 0,1 % atomique) dans un diagramme d'état ternaire Au-Pt-Pd,
l'alliage Au-Pt-Pd médical comprend une quantité totale de 0 ppm ou plus et 200 ppm ou moins de Ag, Co, Cr, Fe, Ir, Mg, Ni, Rh, Ru, Si, Sn et Ti en tant qu'impuretés inévitables et
les éléments additifs et éléments d'impuretés inévitables remplacent une partie de Au dans l'alliage Au-Pt-Pd,
**caractérisé en ce que**
dans une structure métallique sur une coupe transversale quelconque,
avec une composition d'un alliage Au-Pt-Pd en phase mère comme critère, au moins une phase parmi une phase riche en Au qui est une phase d'alliage ayant une teneur en Au supérieure de 4 % atomique ou plus à celle de la phase mère et une phase riche en Pt qui est une phase d'alliage ayant une teneur en Pt supérieure de 4 % atomique ou plus à celle de la phase mère est distribuée et un total d'un rapport de surface de la phase riche en Au et d'un rapport de surface de la phase riche en Pt est de 1,5 % ou plus 25,4 % ou moins, et
l'alliage Au-Pt-Pd médical présente une susceptibilité magnétique en volume de -32 ppm ou plus et 60 ppm ou moins et un module de Young de 100 GPa ou plus,
dans lequel le rapport de surface se réfère à un rapport basé sur le champ visuel d'observation d'une structure matérielle sur une coupe transversale arbitraire et est déterminé comme décrit dans la description, la susceptibilité magnétique en volume est déterminée avec une balance magnétique comme décrit dans la description et le module de Young est déterminé avec un testeur de traction comme décrit dans la description.

2. Alliage Au-Pt-Pd médical selon la revendication 1, qui présente une composition d'alliage comprise dans la plage à l'intérieur d'un polygone (A1-A2-B3-B4) entouré de lignes droites reliées au niveau du point A1 (Au : 53 % atomique, Pt : 4 % atomique, et Pd : 43 % atomique), du point A2 (Au : 70 % atomique, Pt : 4 % atomique, et Pd : 26 % atomique), du point B3 (Au : 70 % atomique, Pt : 20 % atomique, et Pd : 10 % atomique), et du point B4 (Au : 55 % atomique, Pt : 35 % atomique, et Pd : 10 % atomique) dans le diagramme d'état ternaire Au-Pt-Pd.

3. Alliage Au-Pt-Pd médical selon la revendication 1, qui présente une composition d'alliage dans la plage à l'intérieur d'un polygone (A1-C2-C3-C4) entouré de lignes droites reliées au niveau du point A1 (Au : 53 % atomique, Pt : 4 % atomique, et Pd : 43 % atomique), du point C2 (Au : 60 % atomique, Pt : 4 % atomique, et Pd : 36 % atomique), du point C3 (Au : 62 % atomique, Pt : 12 % atomique, et Pd : 26 % atomique), et du point C4 (Au : 54 % atomique, Pt : 20 % atomique, et Pd : 26 % atomique) dans le diagramme d'état ternaire Au-Pt-Pd.

4. Procédé destiné à produire l'alliage Au-Pt-Pd médical défini selon l'une quelconque des revendications 1 à 3, comprenant les étapes destinées à :
fondre et couler un alliage mère de l'alliage Au-Pt-Pd ;
chauffer l'alliage mère à une température de 1000°C ou supérieure et 1200°C ou inférieure pour mettre en oeuvre un traitement d'homogénéisation ;
soumettre l'alliage mère traité par homogénéisation à un travail plastique ;
soumettre l'alliage travaillé de manière plastique à un traitement de solution par chauffage à 1000°C ou supérieur et 1200°C ou inférieur pendant 1 à 24 heures ; et
mettre en oeuvre un traitement thermique de vieillissement dans lequel l'alliage est chauffé à 400 à 800°C pour commander une structure métallique.

5. Procédé destiné à produire un alliage Au-Pt-Pd médical selon la revendication 4, comprenant l'étape de soumission de l'alliage mère traité par solution à un travail plastique avant le traitement thermique de vieillissement.

6. Dispositif médical comprenant l'alliage Au-Pt-Pd médical selon l'une quelconque des revendications 1 à 3.

7. Dispositif médical selon la revendication 6, dans lequel le dispositif médical est un parmi un stent, un cathéter, une bobine d'embolisation, un clip d'embolisation et un fil-guide.
